# FASCICULE DE BREVET EUROPEEN

(11) **EP 0 797 985 B1**
(45) Date de publication et mention de la délivrance du brevet: **03.02.1999**
(21) Numéro de dépôt: 97400605.8
(22) Date de dépôt: 18.03.1997
(51) Int. Cl.: A61K 7/48

(54) **Utilisation d'un extrait d'au moins une iridacée dans le traitement des rides**
Verwendung eines Extrakts mindestens einer Iridacea bei der Behandlung von Falten
Use of an extract of at least one iridaceae in the treatment of skin wrinkles

(30) Priorité: 27.03.1996 FR 9603817
(43) Date de publication de la demande: 01.10.1997
(73) Titulaire: L'OREAL, 75008 Paris (FR)
(72) Inventeur: Breton, Lionel, 78000 Versailles (FR); de Lacharriere, Olivier, 75015 Paris (FR); Martin, Richard, 37210 Rochecorbon (FR)
(74) Mandataire: Tezier Herman, Béatrice

(56) Documents cités:
- WO-A-97/09056
- DATABASE WPI Week 8717 Derwent Publications Ltd., London, GB; AN 87-118803 XP002021294 "Anti-ageing agent for treatment of rough skin - contains extract and/or solid material from iris etc" & JP 62 061 924 A (SHISEIDO) , 18 Mars 1987
- DATABASE WPI Week 9250 Derwent Publications Ltd., London, GB; AN 92-410114 XP002021295 "Skin external moisture-retaining agent - contains extract from saffron extracted with e. g. water or ethanol" & JP 04 305 519 A (TAISHO PHARM) , 28 Octobre 1992
- DATABASE WPI Week 9601 Derwent Publications Ltd., London, GB; AN 96-006882 XP002021296 "Cosmetics for maintaining skin moisture - comprise pptes. obtd. by extracting Gardenia jasminoides or Crocus sativum with water, treating extract and adjusting pH" & JP 07 285 845 A (POLA CHEM) , 31 Octobre 1995
- DATABASE WPI Week 9530 Derwent Publications Ltd., London, GB; AN 95-228633 XP002021297 "Cell activator for preventing skin ageing - comprises Belamcanda chinensis L., belonging to Iridaceae or its dried roots extracts with e. g. water" & JP 07 138 179 A (ICHIMARU PHARCOS) , 30 Mai 1995
- DATABASE WPI Week 8652 Derwent Publications Ltd., London, GB; AN 86-341883 XP002021298 "External application agent for treating rough skin - contains pyridoxine or its deriv. and powder or extract of iris root" & JP 61 254 510 A (SHISEIDO) , 12 Novembre 1986

## Description

La présente invention se rapporte à l'utilisation d'une quantité efficace d'au moins un extrait d'au moins une Iridacée dans une composition cosmétique ou pour la préparation d'une composition pharmaceutique, pour relâcher et/ou relaxer le tissu cutané et/ou sous-cutané, notamment en vue de traiter les rides et les ridules de la peau.

Les femmes, voire même les hommes, ont tendance actuellement à vouloir paraître jeunes le plus longtemps possible et cherchent par conséquent à estomper les marques du vieillissement de la peau, qui se traduisent notamment par des rides et des ridules. A ce sujet, la publicité et la mode font état de produits destinés à garder le plus longtemps possible une peau éclatante et sans rides, marques d'une peau jeune, d'autant plus que l'aspect physique induit sur le psychisme et/ou sur le moral. Or, il est important de se sentir physiquement et moralement jeune.

Jusqu'à présent, on traitait les rides et les ridules à l'aide de produits cosmétiques contenant des actifs agissant sur la peau, par exemple en l'hydratant ou en améliorant son renouvellement cellulaire ou encore en favorisant la synthèse du collagène qui compose le tissu cutané. Mais, à ce jour, on ne sait pas agir sur les rides en intervenant sur les éléments musculaires présents dans la peau.

Il est connu que les muscles peauciers du visage sont sous le contrôle des afférences nerveuses motrices du nerf facial et que, par ailleurs, les cloisons interlobulaires de l'hypoderme contiennent en leur sein des fibres qui constituent un tissu musculaire strié (panniculus carnosus). D'autre part, il est également connu qu'une sous-population de fibroblastes du derme, que l'on appelle myofibroblastes, présente des caractéristiques communes avec le tissu musculaire.

La demanderesse a notamment observé, dans certaines situations pathologiques et thérapeutiques, le rôle joué sur les rides du visage par les nerfs contrôlant l'ensemble de ce tissu musculaire. Ainsi, dans les atteintes du nerf facial, dans lesquelles la transmission de l'influx nerveux est interrompue et/ou amoindrie, on assiste dans le territoire d'innervation à une paralysie des muscles du visage. Cette paralysie faciale se traduit, entre autres signes cliniques, par une atténuation, voire une disparition des rides.

A l'inverse, dans les états d'hypercontraction musculaire de la face, la demanderesse a constaté une accentuation des rides du visage. De plus, elle a également observé une accentuation des rides du visage dans les états d'hypertonie musculaire de la maladie de Parkinson et des effets secondaires induits par les neuroleptiques.

Par ailleurs, il a été montré que la toxine botulique, utilisée à l'origine pour traiter les spasmes, pouvait agir sur les états de spasticité musculaire (voir A. Blitzer et al., Arch. Otolaryngol. Head Neck Surg., 1993, 119, pages 1018 à 1022) et sur les rides de la glabelle qui sont les rides inter sourcilières (voir J.D. Carruters et al., J. Dermatol. Surg. Oncol., 1992, 18, pages 17 à 21). En conséquence, il est possible d'agir par une action pharmacologique sur la composante nerveuse des rides.

Dans le système nerveux périphérique, la jonction entre un nerf et un muscle constitue la plaque neuro-musculaire, en amont de laquelle se trouve la voie nerveuse afférente appelée motoneurone. Par ailleurs, les membranes cellulaires de chaque fibre nerveuse comportent de nombreux canaux ioniques, et notamment des canaux chlore, aptes à laisser traverser l'élément correspondant sous forme ionique, et dans le cas des canaux chlore sous forme de chlorure. A ces canaux, sont associés des récepteurs neuronaux. Les récepteurs neuronaux associés en périphérie aux canaux chlore sont notamment des récepteurs pour la glycine (les récepteurs glycine-strychnine sensibles) et des récepteurs pour le GABA de type A (les récepteurs GABA-A)(GABA = Acide γ-aminobutirique).

On sait qu'il est possible de diminuer l'excitabilité du motoneurone par divers agents pharmacologiques agissant sur les récepteurs glycine-strychnine sensibles ou sur les récepteurs GABA-A du système nerveux périphérique (voir W. Sieghart, Trends in Pharmacological Science, décembre 1992, vol. 131, pages 446 à 450). Ainsi, on peut moduler l'excitabilité du motoneurone par exemple par la glycine ou l'acide gamma amino-butyrique (GABA).

L'activation de ces récepteurs ouvre les canaux chlore et conduit à l'entrée d'ions chlorure, ce qui aboutit à une augmentation des ions chlorure dans les cellules de la fibre nerveuse et donc à une hyperpolarisation des motoneurones qui deviennent par voie de conséquence moins excitables. Cette diminution d'excitabilité du motoneurone entraîne une moindre stimulation de la fibre musculaire, provoquant ainsi son relâchement.

Après de nombreux tests cliniques, la demanderesse a pu déterminer que les fibres musculaires contractiles, en particulier des fibres musculaires striées, qui se trouvent sous le contrôle direct de l'influx neuro-moteur, jouaient un rôle essentiel dans la pathogénie des rides et que la modulation de l'influx neuro-moteur atténuait non seulement les rides mais également les ridules et avait aussi un effet de "lissage" sur le microrelief cutané. Elle a aussi trouvé que les tissus cutané et sous-cutané comportaient des récepteurs associés aux canaux chlore, ce qui, jusqu'à présent, n'avait pas été envisagé. Elle a donc trouvé que l'on pouvait agir sur ces canaux pour relâcher ou relaxer ces tissus, et ainsi diminuer les rides et les ridules.

Personne n'avait, jusqu'à ce jour, établi un lien entre les canaux chlore des fibres nerveuses et les rides, et n'avait trouvé que l'on pouvait traiter les rides en agissant sur les canaux chlore par activation des récepteurs se trouvant dans ou au voisinage de ces canaux. Les substances qui peuvent activer les récepteurs des canaux chlore et donc entraîner l'entrée de chlorure dans les cellules, sont appelées substances agonistes.

Il existe plusieurs récepteurs associés au canal chlore. Il s'agit notamment des récepteurs glycine-strychnine sensibles et des récepteurs GABA-A, ces derniers comportant eux-mêmes plusieurs sous-unités constituées par le site GABA, le site benzodiazépine, un type de site stéroïde et le site aux barbituriques. Toutes les substances agissant comme agonistes de ces récepteurs ou sites peuvent être utilisées pour relâcher ou relaxer les tissus cutané et/ou sous-cutané conformément à l'invention.

Pour qu'une substance soit reconnue comme un agoniste des récepteurs des canaux chlore, elle doit répondre aux deux caractéristiques suivantes :
- pouvoir se fixer sélectivement sur au moins un des différents récepteurs associés au canal chlore ;
- montrer un effet de relaxation sur un tissu musculaire contracté.

La première caractéristique, qui consiste en la possibilité de se fixer sur un récepteur associé à un canal chlore, ne permet pas de distinguer une activité agoniste d'une activité antagoniste, mais elle permet de définir une affinité potentielle pour le récepteur.

La seconde caractéristique permet de sélectionner les agonistes. L'activité agoniste de la substance étudiée peut être mise en évidence par l'effet de relaxation qu'elle produit sur un tissu musculaire qui a été préalablement contracté par une substance antagoniste des canaux chlore. Comme substance antagoniste du canal chlore, on peut choisir les substances connues comme telles, et notamment les substances suivantes : bicuculline, strychnine, terbutyl-bicyclo-phosphorothionate et picrotoxine.

De manière surprenante la demanderesse a montré qu'un extrait d'au moins une Iridacée répondait aux critères d'agoniste des récepteurs des canaux chlore ci-dessus définis.

Il a été décrit dans la demande de brevet japonais n° JP-A-60-201249 l'utilisation d'extrait d'Iridacées pour le traitement de la peau rugueuse par le biais de l'activité dilatatrice des vaisseaux sanguins de la peau.

De même, il a été décrit dans la demande de brevet japonais n° JP-A-04-305519, un agent cosmétique ayant un haut pouvoir de rétention de l'hydratation et un bon touché, contenant un extrait d'Iridacées.

Dans la demande de brevet Japonais n° JP-A-07-285845, il est décrit un agent cosmétique de maintien de l'hydratation, contenant un extrait de *Gardenia jasminoides* ou de *Crocus sativum*.

Dans la demande de brevet Japonais n° JP-A-07-138179, il est décrit un activateur cellulaire comprenant un extrait de *Belamcanda chinensis*.

La présente invention se rapporte à l'utilisation, dans une composition cosmétique ou pour la préparation d'une composition pharmaceutique, d'une quantité efficace d'un extrait d'au moins une Iridacée pour relaxer et/ou relâcher le tissu cutané et/ou sous-cutané.

Cette utilisation s'avère particulièrement efficace pour diminuer les rides et ridules.

L'invention a pour objet l'utilisation d'une quantité efficace d'un extrait d'au moins une Iridacée pour diminuer les rides et/ou les ridules.

Plus particulièrement la relaxation et/ou le relâchement du tissus cutané et/ou sous-cutané est un relâchement ou une relaxation musculaire.

La composition contenant l'extrait peut être appliquée soit par voie locale, c'est-à-dire par voie topique, ou par injection sous-cutanée et/ou intradermique, soit par voie systémique ou générale, c'est-à-dire par voie orale et/ou par injection intramusculaire.

L'invention a aussi pour objet un procédé de traitement cosmétique des rides et/ou des ridules, consistant à appliquer par voie topique, à injecter ou à ingérer une composition comprenant une quantité efficace d'un extrait d'au moins une Iridacée.

L'extrait d'au moins une Iridacée peut être tout extrait préparé à partir de matériel végétal issu de la famille des Iridacées.
La composition peut contenir un extrait d'au moins une Iridacée obtenu à partir de matériel végétal issu de plante entière cultivée *in vivo* ou issu de culture *in vitro.*
La pression de sélection imposée par les conditions physico-chimiques lors de la croissance des cellules végétales *in vitro* permet d'obtenir un matériel végétal standardisé et disponible tout au long de l'année contrairement aux plantes cultivées *in vivo.*

Par culture *in vitro,* on entend l'ensemble des techniques connues de l'homme du métier qui permet de manière artificielle l'obtention d'un végétal ou d'une partie d'un végétal.
On peut ainsi utiliser par exemple selon l'invention un extrait de racines d'au moins une Iridacée cultivées *in vitro* ou encore un extrait de cellules indifférenciées d'au moins une Iridacée.

De préférence, on utilise un extrait obtenu à partir de matériel végétal cultivé *in vitro* et encore plus préférentiellement un extrait obtenu à partir de cellules indifférenciées cultivées *in vitro.*

Par cellules végétales indifférenciées, on entend toute cellule végétale ne présentant aucun des caractères d'une spécialisation particulière et capable de vivre par elle-même et non en dépendance avec d'autres cellules. Ces cellules végétales indifférenciées sont éventuellement aptes, sous l'effet d'une induction, à toute différenciation conforme à leur génome.
Selon la méthode de culture choisie, et en particulier selon le milieu de culture choisi, il est possible d'obtenir, à partir d'un même explant, des cellules végétales indifférenciées présentant des caractères différents.
La famille des Iridacées (ou Iridées) compte environ 750 espèces.
Les plantes de la famille des Iridacées sont surtout utilisées pour leur propriétés aromatiques et ornementales.
Parmi les genres d'lridacées utilisables selon l'invention, on peut citer à titre d'exemple les genres Romulea, Crocus, Iris, Gladiolus, Sisyrinchium ou encore Hermodactylus.

Comme matériel végétal utilisable on peut citer celui provenant d'*Iris germanica,* d'*Iris florentina,* d'*Iris pallida,* de *Crocus versicolor,* de *Romulea bulbucodium* ou encore de *Gladiolus communis.*

Plus particulièrement, selon l'invention, on utilise du matériel végétal issu du genre Iris et préférentiellement du matériel végétal d'*Iris pallida.*

Toute méthode d'extraction connue de l'homme du métier peut être utilisée pour préparer l'extrait d'Iridacée contenu dans la composition.
On peut, en particulier, citer les extraits alcooliques, notamment éthanoliques ou encore hydroalcooliques.

On peut également utiliser un extrait d'Iridacée préparé par la méthode décrite dans la demande de brevet français n° 95-02379 déposée par la demanderesse. Ainsi, dans une première étape on broie le matériel végétal dans une solution aqueuse à froid, dans une deuxième étape les particules en suspension sont éliminées de la solution aqueuse issue de la première étape, et dans une troisième étape on stérilise la solution aqueuse issue de la deuxième étape. Cette solution aqueuse correspond à l'extrait.
D'autre part, la première étape peut avantageusement être remplacée par une opération de congélation simple des tissus végétaux (par exemple à -20°C), suivie d'une extraction aqueuse reprenant les deuxième et troisième étapes ci-dessus décrites.

Un exemple de préparation d'extrait d'Iridacée utilisable selon l'invention est donné par ailleurs dans les exemples.

La quantité efficace d'extrait d'Iridacée contenue dans la composition est bien entendu fonction de l'effet recherché et peut donc varier dans une large mesure.

Pour donner un ordre de grandeur, si la composition est une composition cosmétique, elle peut contenir un extrait d'au moins une Iridacée en une quantité représentant de 0,01% à 20% du poids total de la composition et préférentiellement en une quantité représentant de 0,1% à 5% du poids total de la composition.

Pour donner un ordre de grandeur, si la composition est une composition pharmaceutique, elle peut contenir un extrait d'au moins une Iridacée en une quantité représentant de 0,01% à 30% du poids total de la composition et préférentiellement en une quantité représentant de 0,1% à 10% du poids total de la composition.

Les compositions peuvent se présenter sous toutes les formes galéniques normalement utilisées pour une application topique, injectable ou orale.

Les quantités des différents constituants des compositions sont celles classiquement utilisées dans les domaines considérés et sont appropriées à leur forme galénique.
Pour une application topique, les compositions comprennent un milieu compatible avec la peau. Ces compositions peuvent se présenter notamment sous forme de solutions aqueuses, alcooliques ou hydroalcooliques, de gels, d'émulsions eau-dans-huile ou huile-dans-eau ayant l'aspect d'une crème ou d'un gel, de microémulsions, d'aérosols, ou encore sous forme de dispersions vésiculaires contenant des lipides ioniques et/ou non ioniques. Ces formes galéniques sont préparées selon les méthodes usuelles des domaines considérés.

Ces compositions à application topique peuvent constituer notamment une composition de protection, de traitement ou de soin cosmétique ou pharmaceutique pour le visage, pour le cou, pour les mains ou pour le corps, (par exemple crèmes de jour, crèmes de nuit, crèmes ou huiles solaires, laits corporels), une composition de maquillage (par exemple fond de teint) ou une composition de bronzage artificiel.

Quand la composition est une émulsion, la proportion de corps gras qu'elle contient peut aller de 5 % à 80 % en poids, et de préférence de 5 % à 50 % en poids par rapport au poids total de la composition. Les corps gras et les émulsionnants utilisés dans la composition sous forme d'émulsion sont choisis parmi ceux classiquement utilisés dans le domaine cosmétique ou pharmaceutique.

Comme corps gras utilisables dans l'invention, on peut citer les huiles minérales (vaseline), les huiles végétales (fraction liquide de beurre de karité) et leurs dérivés hydrogénés, les huiles animales, les huiles de synthèse (perhydrosqualène), les huiles siliconées (diméthylpolysiloxane) et les huiles fluorées. Comme autres corps gras, on peut encore citer les alcools gras (alcool cétylique, alcool stéarylique), les acides gras (acide stéarique) et les cires.

Les émulsionnants peuvent être présents, dans la composition, en une proportion allant de 0,3 % à 30 % en poids, et de préférence de 0,5 à 30 % en poids par rapport au poids total de la composition.

De façon connue, les compositions cosmétiques ou pharmaceutiques peuvent contenir également des adjuvants habituels dans les domaines correspondants, tels que les gélifiants hydrophiles ou lipophiles, les conservateurs, les antioxydants, les solvants, les parfums, les charges, les filtres et les matières colorantes. Par ailleurs, ces compositions peuvent contenir des actifs hydrophiles ou lipophiles. Les quantités de ces différents adjuvants ou actifs sont celles classiquement utilisées dans le domaine cosmétique ou pharmaceutique, et par exemple de 0,01 % à 20 % du poids total de la composition. Ces adjuvants ou ces actifs, selon leur nature, peuvent être introduits dans la phase grasse, dans la phase aqueuse et/ou dans des vésicules lipidiques.

Parmi les actifs que peuvent contenir les compositions, on peut notamment citer les actifs ayant un effet sur le traitement des rides ou des ridules, et en particulier les actifs kératolytiques. Par kératolytique, on entend un actif ayant des propriétés desquamantes, exfoliantes ou gommantes, ou un actif capable de ramollir la couche cornée.

Parmi les actifs ayant un effet sur le traitement des rides ou des ridules que peuvent contenir les compositions, on peut en particulier citer les hydroxyacides et les rétinoïdes.

Les hydroxyacides peuvent être par exemple des α-hydroxy-acides ou des β-hydroxy-acides, qui peuvent être linéaires, ramifiés ou cycliques, saturés ou insaturés. Les atomes d'hydrogène de la chaîne carbonée peuvent, en outre, être substitués par des halogènes, des radicaux halogénés, alkylés, acylés, acyloxylés, alcoxy carbonylés ou alcoxylés ayant de 2 à 18 atomes de carbone.

Les hydroxyacides qui peuvent être utilisés sont notamment les acides glycolique, lactique, malique, tartrique, citrique, hydroxy-2 alcanoïque, mandélique, salicylique, ainsi que leurs dérivés alkylés comme l'acide n-octanoyl-5-salicylique, l'acide n-dodécanoyl-5-salicylique, l'acide n-décanoyl-5-salicylique, l'acide n-octyl-5-salicylique, l'acide n-heptyloxy-5 ou -4-salicylique, l'acide 2-hydroxy-3-méthylbenzoïque, ou encore leurs dérivés alcoxylés comme l'acide 2-hydroxy-3-méthoxybenzoïque.

Les rétinoïdes peuvent être notamment l'acide rétinoïque (all-trans ou 13-cis) et ses dérivés, le rétinol (vitamine A) et ses esters tels que le palmitate de rétinol, l'acétate de rétinol et le propionate de rétinol, ainsi que leurs sels.

Ces actifs peuvent être utilisés en particulier à des concentrations allant de 0,0001% à 5% en poids par rapport au poids total de la composition.
Quand les compositions sont destinées à une application par voie orale, elles peuvent se présenter sous les formes galéniques habituelles dans ce domaine, telles que les comprimés, les gélules, les produits buvables, notamment constitués extemporanément, les granulés, les poudres, dans les excipients habituels pour une telle application.

Quand les compositions sont destinées à être injectées, elles peuvent se présenter sous forme de solutions contenant les excipients habituellement utilisés pour les injections, et par exemple sous forme d'une solution isotonique de chlorure de sodium.

Les exemples et compositions suivants illustrent l'invention. Dans les compositions les proportions indiquées sont des pourcentages en poids, sauf mention contraire.

### Exemple 1 : Préparation d'un extrait d'Iris pallida :

Des cellules indifférenciées d'Iris pallida cultivées in vitro en conditions axéniques sont récupérées après culture en Erlenmeyer ou en fermenteur par filtration sur tamis de 50µm. A 55 g de matière fraîche ainsi obtenue, on ajoute 27,5 ml d'eau déminéralisée. L'ensemble est broyé au Turax à 24000 T/min durant 1 minute à 4°C (bain de glace). Le broyât est centrifugé 15 à 10000 G à 4°C. Le surnageant est filtré à 0,22 µm (filtration stérilisante).
L'extrait ainsi préparé est conservé à 4°C. Il renferme environ 15 g de matière sèche par litre.
Si le matériel végétal est de la plante entière, on ramène la matière fraîche à traiter en fonction du poids sec pour se mettre dans les mêmes conditions d'extraction que pour l'in vitro. Les différentes parties de la plante sont prélevées en fonction du poids relatif de chaque partie de celle-ci. Le traitement à froid permet de geler les activités enzymatiques, la filtration stérilisante évite la dégradation des actifs par les micro-organismes de l'environnement. Enfin, le véhicule eau est compatible avec les récepteurs ex vivo et facilite les formulations cosmétiques ou pharmaceutiques.

### Exemple 2 : mesure de l'affinité de l'extrait d'Iris pallida pour les récepteurs à la glycine et au GABA-A (Récepteur de type A à l'acide γ-aminobutirique) :

L'affinité pour les récepteurs à la glycine a été déterminée selon la méthode décrite par Marvizon et collaborateurs dans Mol. Pharmacol., 1986, 30 : 590-597. L'affinité pour les récepteurs au GABA-A a été déterminée selon la méthode décrite par Snodgrass, S.R., Nature, 1978, 273 : 392.

### Résumé des conditions expérimentales :

### Récepteur à la glycine :

On mesure le déplacement de strychnine tritiée fixée sur le récepteur à la glycine par incubation préalable de 2 nM de strychnine tritiée avec des membranes de moelle épinière de rat pendant 10 minutes à 0°C.
L'extrait d'*Iris pallida* est testé à 1% et 5% de sa concentration initiale.
L'IC₅₀ de la strychnine (dose nécessaire pour déplacer 50 % de la strychnine tritiée fixée au récepteur) est mesurée par déplacement par de la strychnine non marquée.

### Récepteur au GABA-A:

On mesure le déplacement de muscimol tritié fixé sur le récepteur au GABA-A par incubation préalable de 2,5 nM de muscimol tritié avec du cortex cérébral de rat pendant 10 minutes à 4°C.
L'extrait d'*Iris pallida* est testé à 1% et 5% de sa concentration initiale.
L'IC₅₀ du muscimol (dose nécessaire pour déplacer 50 % du muscimol tritié fixé au récepteur) est mesurée par déplacement par du muscimol non marquée.

Résultats : exprimés en pourcentage d'inhibition de fixation du ligand radioactif

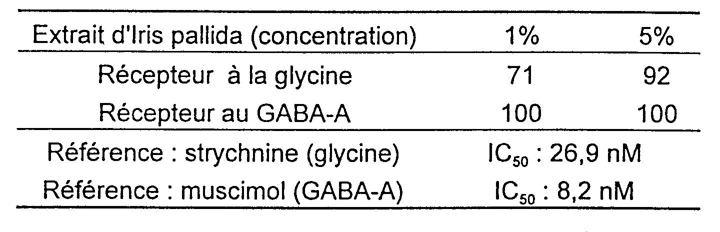

Ces résultats montrent que l'extrait d'*Iris pallida* est un bon ligand des récepteurs à la glycine et au GABA-A.
La relation existant entre ces récepteurs, les canaux chlore et les contractions musculaires fait que l'extrait d'*Iris pallida* est un bon agent antiride.

### Exemple 3 : Mesure de l'effet de l'extrait d'Iris pallida sur le délai d'apparition d'un effet antagoniste des canaux chlore (convulsions) induit par la strychnine chez la souris après administration sous-cutanée.

La mesure est effectuée selon la méthode décrite par Krall et collaborateurs, Epilepsia, 1978, 19, 409-428.
L'extrait d'*Iris pallida* est administré par voie sous-cutanée sous un volume de 10 ml/kg, aux doses désirées. 30 minutes après traitement, une solution de strychnine est injectée par voie sous cutané à la dose de 1 mg/kg. Le véhicule est de l'eau stérile.
On observe l'apparition des convulsions pendant 30 minutes après l'injection de la strychnine. On mesure également le délai d'apparition des convulsions.

Le produit de référence est le Diazépam à 3 mg/kg.

Résultats : Le délai d'apparition des convulsions est exprimé en secondes. Les valeurs données sont des moyennes des délais mesurés par animal dans chaque groupe.

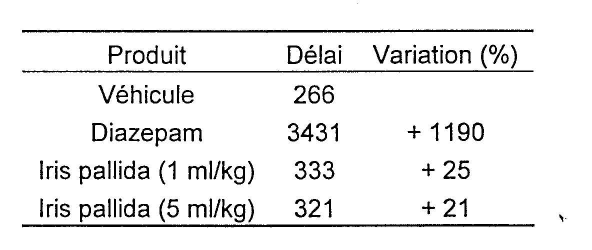

L'extrait d'*Iris pallida* présente, en retardant le temps d'apparition des convulsions, un pouvoir relaxant qui en fait un actif antiride.

### Exemple 4 : exemples de compositions

### Composition 1 : Lotion de soin pour le visage

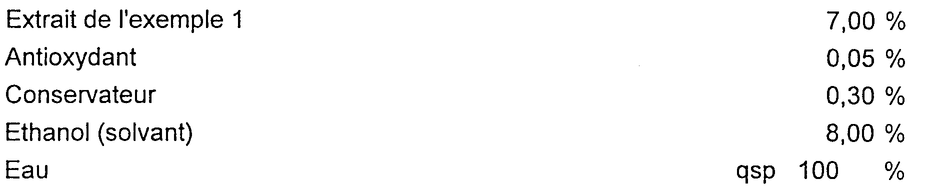

La lotion obtenue agit sur les rides lors d'une utilisation répétée (application biquotidienne pendant un mois).

### Composition 2 : Gel pour le soin du visage

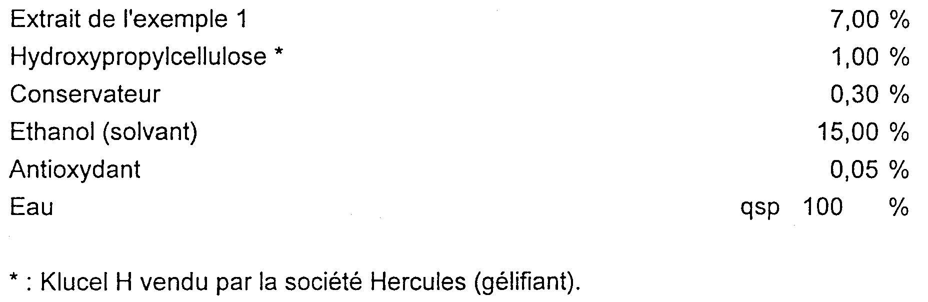

Le gel obtenu agit sur les rides. Il peut être appliqué quotidiennement matin et soir pendant un mois.

### Composition 3 : Crème de soin du visage (émulsion huile-dans-eau)

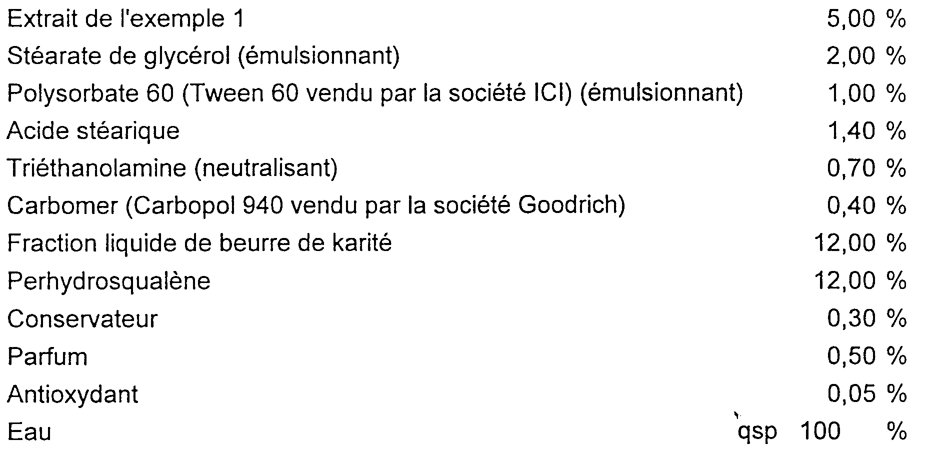

On obtient une crème blanche, onctueuse, qui agit sur les rides et les ridules, et que l'on peut appliquer quotidiennement.

### Composition 4 : Crème de soin du visage (émulsion huile-dans-eau)

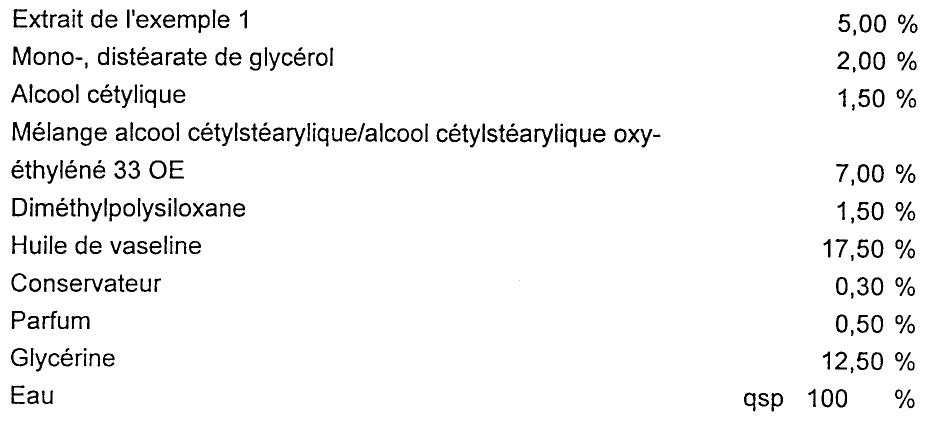

## Revendications

1. Utilisation dans une composition cosmétique ou pour la préparation d'une composition pharmaceutique, d'une quantité efficace d'un extrait d'au moins une Iridacée pour relaxer et/ou relâcher le tissu cutané et/ou sous-cutané.

2. Utilisation selon la revendication 1, pour diminuer les rides et/ou les ridules.

3. Utilisation selon l'une quelconque des revendications précédentes, caractérisée en ce que la composition cosmétique et/ou pharmaceutique est conditionnée sous une forme convenable pour une administration par voie topique, par voie orale et/ou par voie injectable.

4. Utilisation selon l'une quelconque des revendications précédentes, caractérisée en ce que l'extrait est un extrait de plante entière.

5. Utilisation selon l'une quelconque des revendications 1 à 3, caractérisée en ce que l'extrait est un extrait de matériel végétal obtenu par culture *in vitro*.

6. Utilisation selon la revendication 5, caractérisée en ce que le matériel végétal est obtenu par culture *in vitro* de cellules indifférenciées.

7. Utilisation selon l'une quelconque des revendications précédentes, caractérisée en ce que ledit extrait est un extrait de matériel végétal provenant d'Iridacées d'un genre choisi parmi Romulea, Crocus, Iris, Gladiolus, Sisyrinchium ou Hermodactylus.

8. Utilisation selon la revendication 7, caractérisée en ce que ledit extrait est un extrait de matériel végétal provenant d'Iris.

9. Utilisation selon la revendication 8, caractérisée en ce que ledit extrait est un extrait d'*Iris pallida*.

10. Utilisation selon l'une quelconque des revendications précédentes, caractérisée en ce que dans la composition cosmétique ledit extrait d'Iridacée est utilisé en une quantité représentant de 0,01 % à 20 % du poids total de la composition et de préférence de 0,1 % à 5 % du poids total de la composition.

11. Utilisation selon l'une quelconque des revendications 1 à 9, caractérisée en ce que dans la composition pharmaceutique ledit extrait d'Iridacée est utilisé en une quantité représentant de 0,01 % à 30 % du poids total de la composition et préférentiellement en une quantité représentant de 0,1 % à 10 % du poids total de la composition.

12. Procédé de traitement cosmétique des rides et/ou des ridules, consistant à appliquer par voie topique, à injecter ou à ingérer une composition comprenant une quantité efficace d'au moins un extrait d'une Iridacée.

## Claims

1. Use, in a cosmetic composition or for the preparation of a pharmaceutical composition, of an effective quantity of an extract of at least one Iridaceae for relaxing and/or loosening the cutaneous and/or subcutaneous tissue.

2. Use according to Claim 1, for reducing normal and/or small wrinkles.

3. Use according to any one of the preceding claims, characterized in that the cosmetic and/or pharmaceutical composition is packaged in a form suitable for administration via the topical route, the oral route and/or the injectable route.

4. Use according to any one of the preceding claims, characterized in that the extract is a whole plant extract.

5. Use according to any one of Claims 1 to 3, characterized in that the extract is an extract of plant material obtained by *in vitro* culture.

6. Use according to Claim 5, characterized in that the plant material is obtained by *in vitro* culture of undifferentiated cells.

7. Use according to any one of the preceding claims, characterized in that the said extract is an extract of plant material obtained from Iridaceae of a genus chosen from Romulea, Crocus, Iris, Gladiolus, Sisyrinchium or Hermodactylus.

8. Use according to Claim 7, characterized in that the said extract is an extract of plant material obtained from Iris.

9. Use according to Claim 8, characterized in that the said extract is an *Iris pallida* extract.

10. Use according to any one of the preceding claims, characterized in that in the cosmetic composition, the said Iridaceae extract is used in a quantity representing from 0.01% to 20% of the total weight of the composition and preferably from 0.1% to 5% of the total weight of the composition.

11. Use according to any one of Claims 1 to 9, characterized in that in the pharmaceutical composition, the said Iridaceae extract is used in a quantity representing from 0.01% to 30% of the total weight of the composition and preferably in a quantity representing from 0.1% to 10% of the total weight of the composition.

12. Process for the cosmetic treatment of normal and/or small wrinkles, consisting in applying by the topical route, in injecting or in ingesting a composition comprising an effective quantity of at least one extract of an Iridaceae.

## Patentansprüche

1. Verwendung einer wirksamen Menge eines Extrakts mindestens einer Iridacea in einer kosmetischen Zusammensetzung oder für die Herstellung einer pharmazeutischen Zusammensetzung zur Relaxation und/oder Entspannung des Hautgewebes und/oder Unterhautgewebes.

2. Verwendung nach Anspruch 1 für die Verminderung von Falten und/oder Fältchen.

3. Verwendung nach einem der vorhergehenden Ansprüche, dadurch gekennzeichnet, daß die kosmetische und/oder pharmazeutische Zusammensetzung in einer Form konfektioniert ist, die für eine Verabreichung auf topischem Wege oder oralem Wege und/oder durch Injizieren geeignet ist.

4. Verwendung nach einem der vorhergehenden Ansprüche, dadurch gekennzeichnet, daß der Extrakt ein Extrakt ganzer Pflanzen ist.

5. Verwendung nach einem der Ansprüche 1 bis 3, dadurch gekennzeichnet, daß der Extrakt ein Extrakt eines durch *in vitro*-Kultivierung erhaltenen pflanzlichen Materials ist.

6. Verwendung nach Anspruch 5, dadurch gekennzeichnet, daß das pflanzliche Material durch *in vitro*-Kultivierung von undifferenzierten Zellen erhalten wird.

7. Verwendung nach einem der vorhergehenden Ansprüche, dadurch gekennzeichnet, daß der Extrakt ein Extrakt eines pflanzlichen Materials ist, das von Iridaceen einer Gattung stammt, die unter Romulea, Crocus, Iris, Gladiolus, Sisyrinchium und Hermodactylus ausgewählt wird.

8. Verwendung nach Anspruch 7, dadurch gekennzeichnet, daß der Extrakt ein Extrakt eines pflanzlichen Materials ist, das von der Gattung Iris stammt.

9. Verwendung nach Anspruch 8, dadurch gekennzeichnet, daß der Extrakt ein Extrakt von *Iris pallida* ist.

10. Verwendung nach einem der vorhergehenden Ansprüche, dadurch gekennzeichnet, daß der Iridacea-Extrakt in der kosmetischen Zusammensetzung in einer Menge von 0,01 bis 20 % des Gesamtgewichts der Zusammensetzung und vorzugsweise 0,1 bis 5 % des Gesamtgewichts der Zusammensetzung verwendet wird.

11. Verwendung nach einem der Ansprüche 1 bis 9, dadurch gekennzeichnet, daß der Iridacea-Extrakt in der pharmazeutischen Zusammensetzung in einer Menge von 0,01 bis 30 % des Gesamtgewichts und vorzugsweise in einer Menge von 0,1 bis 10 % des Gesamtgewichts der Zusammensetzung verwendet wird.

12. Verfahren zur kosmetischen Behandlung von Falten und/oder Fältchen, das darin besteht, eine Zusammensetzung, die eine wirksame Menge mindestens eines Iridacea-Extrakts enthält, auf topischem Wege, durch Injizieren oder durch Einnehmen anzuwenden.
